# EUROPEAN PATENT SPECIFICATION

(11) **EP 2 372 362 B1**
(45) Date of publication and mention of the grant of the patent: **27.03.2013**
(21) Application number: 11160375.9
(22) Date of filing: 30.03.2011
(51) Int. Cl.: G01N 33/574, G06F 19/00

(54) **Method for judging susceptibility of cancer cells to anthracycline anticancer agent and computer program**
Verfahren zur Beurteilung der Empfindlichkeit von Krebszellen gegenüber einem Anthracyclin-Antitumormittel und Computerprogramm
Procédé de jugement de la susceptibilité des cellules cancéreuses à un agent anti-cancer d'anthracycline et programme informatique

(30) Priority: 31.03.2010 JP 2010081423
(43) Date of publication of application: 05.10.2011
(73) Proprietor: Sysmex Corporation, Kobe-shi, Hyogo 651-0073 (JP)
(72) Inventor: Ohyama, Tomoko, Kobe-shi Hyogo 651-0073 (JP); Shibayama, Masaki, Kobe-shi Hyogo 651-0073 (JP); Ishihara, Hideki, Kobe-shi Hyogo 651-0073 (JP); Yoshida, Tomokazu, Kobe-shi Hyogo 651-0073 (JP); Gohda, Keigo, Kobe-shi Hyogo 651-0073 (JP)
(74) Representative: Paemen, Liesbet R.J.

(56) References cited:
- EP-A2- 1 921 170
- KALININA E V ET AL: "Expression of genes for redox-dependent glutathione S-transferase isoforms GSTP1-1 and GSTA4-4 in tumor cell during the development doxorubicin resistance", BULLETIN OF EXPERIMENTAL BIOLOGY AND MEDICINE, KLUWER ACADEMIC PUBLISHERS-PLENUM PUBLISHERS, NE, vol. 143, no. 3, 1 March 2007 (2007-03-01) , pages 328-330, XP019523726, ISSN: 1573-8221, DOI: 10.1007/S10517-007-0102-2
- ANDREW HARBOTTLE ET AL: "Role of glutathioneS-transferase P1, P-glycoprotein and multidrug resistance-associated protein 1 in acquired doxorubicin resistance", INTERNATIONAL JOURNAL OF CANCER, vol. 92, no. 6, 15 June 2001 (2001-06-15), pages 777-783, XP55005939, ISSN: 0020-7136, DOI: 10.1002/ijc.1283
- ARAI T ET AL: "Association of GSTP1 expression with resistance to docetaxel and paclitaxel in human breast cancers", EUROPEAN JOURNAL OF SURGICAL ONCOLOGY, LONDON, GB, vol. 34, no. 7, 1 July 2008 (2008-07-01), pages 734-738, XP022731244, ISSN: 0748-7983, DOI: 10.1016/J.EJSO.2007.07.008 [retrieved on 2008-06-14]
- KIM S J ET AL: "Determination of the specific activity of CDK1 and CDK2 as a novel prognostic indicator for early breast cancer", ANNALS OF ONCOLOGY, KLUWER, DORDRECHT, NL, vol. 19, no. 1, 1 January 2008 (2008-01-01), pages 68-72, XP008091754, ISSN: 0923-7534, DOI: 10.1093/ANNONC/MDM358

## Description

### FIELD OF THE INVENTION

The present invention relates to a method for judging susceptibility of breast cancer cells to an anthracycline anticancer agent based on the expression level of GST-π and a computer program.

### BACKGROUND

One of the therapies for cancer treatment is a chemotherapy regimen and various types of anticancer agents have been used. However, effective anticancer agents vary depending on the type of cancer and patient's individual difference. There are many cases where, as for an anticancer agent, there is overlap between therapeutic range and harmful range. Accordingly, there is a danger that the possibility of cancer recurrence is increased when an ineffective anticancer agent is administered. In addition, a patient's strength is lowered by administration of the ineffective anticancer agent. Therefore, there is a danger that even if another effective anticancer agent is then administered to the patient, its efficacy cannot be sufficiently demonstrated. From the foregoing, there is demand for development of a device for predicting drug responsiveness to specify an effective anticancer agent accurately prior to administration in order to secure safety and attain effectiveness.

Conventionally, a method which includes contacting various anticancer agents with cancer cells isolated from a patient, and on the basis of growth suppression etc: of the cancer cells as the indicator, specifying an anticancer agent estimated to be effective against the cancer cells has been used in examination of the susceptibility of anticancer agents to cancer. However, there are cases where examination results of such trial-and-error method are not sufficiently indicative of clinical effects. In addition, large amounts of cancer cells are necessary in the treatment of anticancer agents. Thus, there are problems, such as cancer recurrence due to administration of a less effective anticancer agent and burden on patients due to extraction of large amounts of cancer cells.

The anthracycline anticancer agent is an anticancer agent for inducing apoptosis by inhibiting the rebinding of DNA in topoisomerase II. The anthracycline anticancer agent is highly evaluated on clinical studies and is used widely in chemotherapy of breast cancer. However, as with other anticancer agents, the anthracycline anticancer agent can cause serious side effects such as cardiotoxicity and leukopenia. Therefore, it is very important to establish the method for judging susceptibility of cancer cells to an anthracycline anticancer agent.

Glutathione-S-transferase π (GST-π) is a protein of about 22.5 kDa molecular weight encoded by a GSTP1 gene of enzymes belonging to a human glutathione-S-transferase (GST) family. GST-π is involved in the process of catalyzing the conjugation of reduced glutathione (GSH) with an anticancer agent to reduce the toxicity of the anticancer agent. It is known that, generally, cancer cells exhibiting high expression of GST-π are highly resistant to anticancer agents as compared with cancer cells exhibiting low expression of GST-π. For example, the fact that, generally, cancer cells exhibiting high expression of GST-π are highly resistant to the anthracycline anticancer agent as compared with cancer cells exhibiting low expression of GST-π is reported in Andrew H., et al. (ROLE OF GLUTATHIONE S-TRANSFERASE P1, P-GLYCOPROTEIN AND MULTIDRUG RESISTANCE-ASSOCIATED PROTEIN 1 IN ACQUIRED DOXORUBICIN RESISTANCE. Int. J. Cancer: 92, 777-783(2001)).

EP1921170 discloses a method (claim 1, 7) as well as a device (claim 13) for determining the efficacy of an anthracycline anticancer agent (eg. doxorubicin: claim 4) comprising determining the expression of Akt in a cancer sample (eg. breast cancer: claim 19 ; fig 12 ; examples 1, 3). High levels of Akt are associated with susceptibility to said anticancer agents.

Kalinina et al (2007. Bulletin Exp Biol Med, 143(3), 328-330) noted that GSP-phi (herein called GSTP1-1) expression is increased in tumour cell lines when resistance to doxorubcine has developed.

Arai et al (2008. EJSO, 734-738) discloses that GSTP1 positive breast tumors show less reduction when treated with doxetaxel or paclitaxel.

Harbottle et al (2001. Int J Cancer, 92, 777-783) discloses that inhibition of GST-phi (GSTP1) chemosensitises tumour cells to doxorubicin.

### SUMMARY OF THE INVENTION

The scope of the present invention is defined solely by the appended claims, and is not affected to any degree by the statements within this summary.

An object of the present invention is to provide a method for judging susceptibility of breast cancer cells to an anthracycline anticancer agent and a computer program which makes a computer execute the method.

As described above, it has been conventionally thought that when cancer cells exhibit high expression of GST-π, the resistance to an anthracycline anticancer agent increases. However, the present inventors have surprisingly found that the breast cancer cells exhibiting high expression of GST-π have high susceptibility of breast cancer cells to an anthracycline anticancer agent and completed the present invention.
(1) A first aspect of the present invention is a method for judging susceptibility of breast cancer cells contained in a biological sample to an anthracycline anticancer agent comprising steps of: measuring expression levels of GST-π of breast cancer cells contained in a biological sample; and judging the susceptibility of breast cancer cells contained in a biological sample to an anthracycline anticancer agent as high when the expression level of GST-π obtained by the measuring process is high.
(2) The method according to (1), wherein the judging step compares the expression level of GST-π obtained by the measuring step with the first threshold value and judges the susceptibility of breast cancer cells contained in a biological sample to an anthracycline anticancer agent as high when the expression level of GST-π is higher than the first threshold value.
(3) The method according to any of (1) or (2), wherein the anthracycline anticancer agent is daunorubicin, doxorubicin, pirarubicin, aclarubicin, epirubicin, oxaunomycin or idarubicin.
(4) The method according to any of (1) to (3), wherein the anthracycline anticancer agent is doxorubicin.
(5) The method according to any of (1) to (4), wherein the measuring step measures specific activity values of CDK1 and CDK2 contained in the biological sample containing breast cancer cells and the judging step compares a value of specific activity value of CDK2 obtained by the measuring step/specific activity value of CDK1 obtained by the measuring step (CDK2 specific activity value/CDK1 specific activity value) with a second threshold value when the expression level of GST-π obtained by the measuring step is high, and the judging step judges the susceptibility of breast cancer cells contained in a biological sample to an anthracycline anticancer agent as high when the value of CDK2 specific activity value/CDK1 specific activity value is higher than the second threshold value.
(6) The method according to (5), wherein the judging step compares the expression level of GST-π obtained by the measuring step with the first threshold value and compares the value of CDK2 specific activity value/CDK1 specific activity value obtained by the measuring step with the second threshold value, when the expression level of GST-π is higher than the first threshold value or when the value of CDK2 specific activity value/CDK1 specific activity value is higher than the second threshold value, the judging step judges the susceptibility of the breast cancer cells contained in a biological sample to an anthracycline anticancer agent as high.
(7) The method according to (5), wherein the judging step judges the susceptibility of breast cancer cells contained in a biological sample to an anthracycline anticancer agent as low when the expression level of GST-π obtained by the measuring step is low and the value of CDK2 specific activity value/CDK1 specific activity value is lower than the second threshold value.
(8) The method according to (7), wherein the judging step compares the expression level of GST-π obtained by the measuring step with the first threshold value and compares the value of CDK2 specific activity value/CDK1 specific activity value with the second threshold value, when the expression level of GST-π is lower than the first threshold value and the value of CDK2 specific activity value/CDK1 specific activity value is lower than the second threshold value, the judging step judges the susceptibility of breast cancer cells contained in a biological sample to an anthracycline anticancer agent as low when the expression level of GST-π obtained by the measuring step is low.
(9) A second aspect of the present invention is a computer program product comprising: a computer readable medium, and software instructions, on the computer readable medium, for enabling a computer to perform operations comprising: acquiring the expression level of GST-π contained in a biological sample containing breast cancer cells; comparing the expression level of GST-π with a first threshold value; judging susceptibility of the breast cancer cells contained in the biological sample to an anthracycline anticancer agent as high when the expression level of GST-π is higher than the first threshold value; and outputting the judged result.
(10) The computer program product according to (9), wherein the operations further comprising: calculating a value of specific activity value of CDK2/specific activity value of CDK1 (CDK2 specific activity value/CDK1 specific activity value) based on the activity value and expression level of CDK1 and the activity value and expression level of CDK2; acquiring the activity value and expression level of CDK1 and the activity value and expression level of CDK2 in the biological sample containing breast cancer cells; and comparing the value of CDK2 specific activity value/CDK1 specific activity value with a second threshold value; wherein a judging operation judges the susceptibility of breast cancer cells contained in a biological sample to an anthracycline anticancer agent as high when the expression level of GST-π is higher than the first threshold value or when the value of CDK2 specific activity value/CDK1 specific activity value is higher than the second threshold value.
(11) The computer program product according to (10), wherein a judging operation judges the susceptibility of breast cancer cells contained in a biological sample to an anthracycline anticancer agent as low when the expression level of GST-π is lower than the first threshold value and the value of CDK2 specific activity value/CDK1 specific activity value is lower than the second threshold value.
(12) The computer program product according to any of (9) to (11), wherein the anthracycline anticancer agent is daunorubicin, doxorubicin, pirarubicin, aclarubicin, epirubicin, oxaunomycin or idarubicin.

According to the present invention, there is provided a method for objectively and accurately judging susceptibility of breast cancer cells to an anthracycline anticancer agent without imposing undue stress on patients. According to the present invention, there is provided a computer program which makes a computer execute the method for judging susceptibility.

According to the method and the computer program of the present invention, the susceptibility of the anthracycline anticancer agent to cancer patients can be judged based on the expression levels of GST-π in the breast cancer cells extracted from the cancer patients. Thus, an effective administration of the anthracycline anticancer agent for the patients can be achieved. Further, an unnecessary administration of the anthracycline anticancer agent can be avoided. Consequently, according to the present invention, the patient's stress due to side effects can be reduced.

### BRIEF DESCRIPTION OF THE DRAWINGS

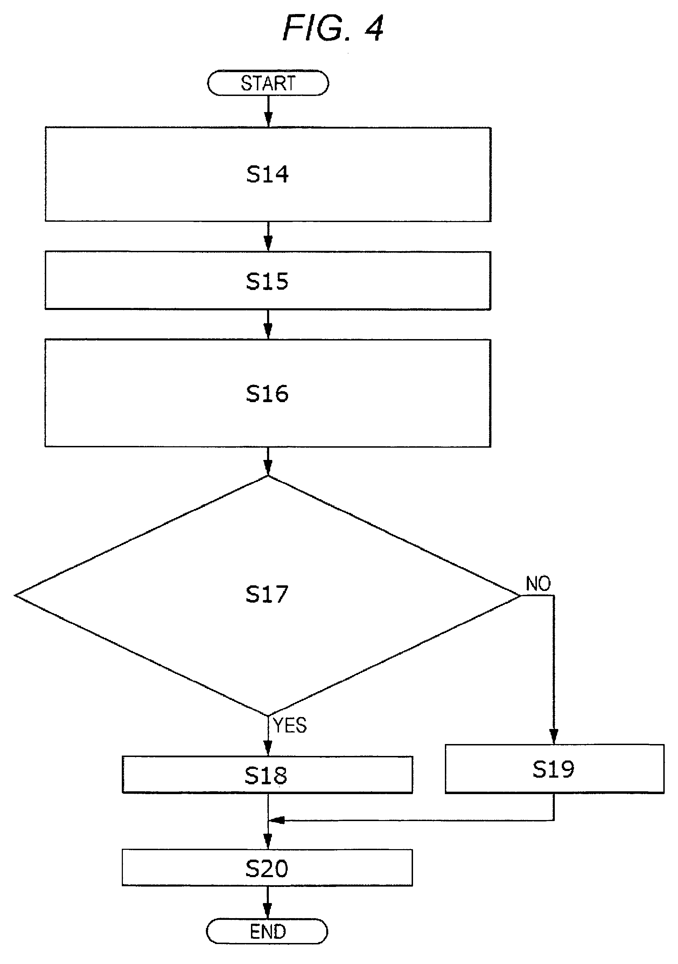

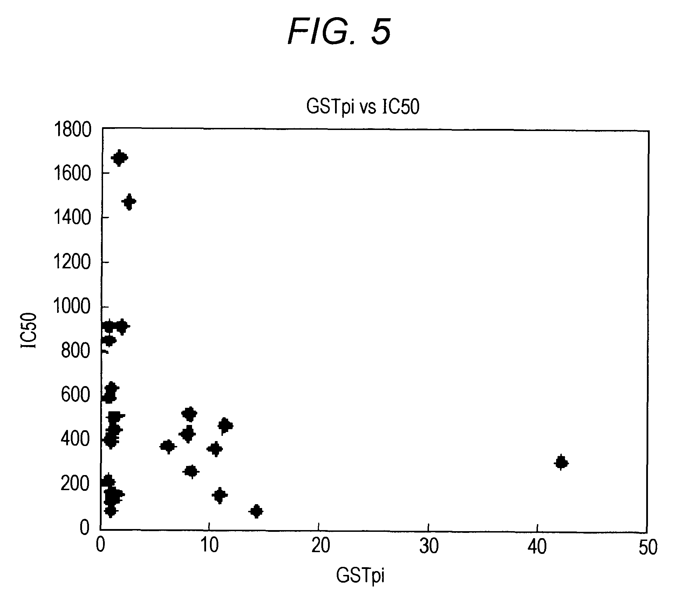

Fig. 1 shows one embodiment of a computer which judges the susceptibility of cancer cells contained in a biological sample to the anthracycline anticancer agent;
Fig. 2 is a judgment flow chart in the computer which judges the susceptibility to the anthracycline anticancer agent as high using the expression levels of GST-π;
   S1: Acquire the expression level of GST-π
   S2: Compare it with a threshold value
   S3: Is the expression level of GST-π more than the threshold value?
   S4: Judge susceptibility as high
   S5: Make the judgment pending
   S6: Output the judged result
Fig. 3 is a judgment flow chart in the computer which judges the susceptibility to the anthracycline anticancer agent as high using the expression levels of GST-π and CDK1 and CDK2 specific activity values;
   S7: Acquire the expression level of GST-π, the activity value and expression level of CDK1, and the activity value and expression level of CDK2
   S8: Calculate a value of CDK2 specific activity value/CDK1 specific activity value
   S9: Compare the expression level of GST-π with a first threshold value and compare the value of CDK2 specific activity value/CDK1 specific activity value with a second threshold value
   S10: Is the expression level of GST-π more than the first threshold value or is the value of CDK2 specific activity value/CDK1 specific activity value more than the second threshold value?
   S11: Judge susceptibility as high
   S12: Make the judgment pending
   S13: Output the judged result
Fig. 4 is a judgment flow chart in the computer which judges the susceptibility to the anthracycline anticancer agent as high or as low using the expression levels of GST-π and the CDK1 and CDK2 specific activity values;
   S14: Acquire the expression level of GST-π, the activity value and expression level of CDK1, and the activity value and expression level of CDK2
   S15: Calculate a value of CDK2 specific activity value/CDK1 specific activity value
   S16: Compare the expression level of GST-π with a first threshold value and compare the value of CDK2 specific activity value/CDK1 specific activity value with a second threshold value
   S17: Is the condition where the expression level of GST-π is less than the first threshold value and the value of CDK2 specific activity value/CDK1 specific activity value is less than the second threshold value satisfied?
   S18: Judge susceptibility as low
   S19: Judge susceptibility as high
   S20: Output the judged result
Fig. 5 is a view showing IC50 results of 29 types of breast cancer cell lines and the expression levels of GST-π in Example 1;

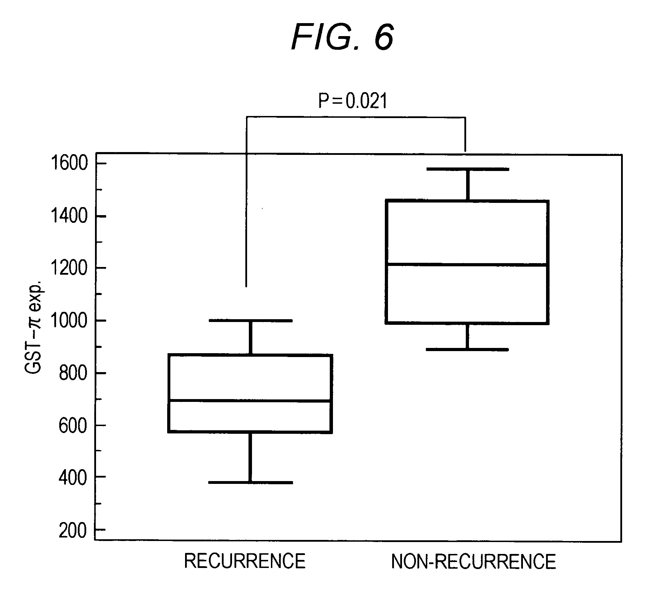

Fig. 6 is a view showing the expression levels of GST-π in a non-recurring specimen and a recurring specimen among 9 clinical specimens in Example 2; and

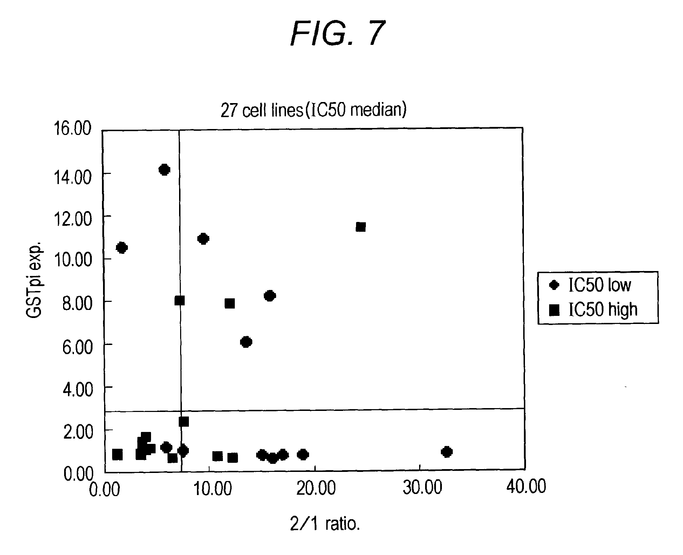

Fig. 7 is a view showing IC50 results of 27 types of breast cancer cell lines, the expression levels of GST-π, and a value of CDK2 specific activity value/CDK1 specific activity value in Example 3.

### DETALLED DESCRIPTION OF THE PREFERRED EMBODIMENTS

Hereinafter, the preferred embodiment of the present invention will be described with reference to the drawings.

In the present embodiment, the cancer cells are breast cancer cells.

In the present embodiment, the cancer patients are breast cancer patients.

In the present embodiment, the biological sample is not particularly limited as long as it is a sample containing a plurality of cells extracted from animals such as humans. Examples thereof include blood, serum, lymph, urine, papilla secretory fluid, and cells and tissues extracted from subjects by surgery or biopsy. Samples obtained by culturing the cells and tissues extracted from subjects may also be used as a biological sample.

In the present embodiment, the anthracycline anticancer agent is not particularly limited as long as it is an anthracycline compound having an anticancer effect. Examples thereof include daunorubicin, doxorubicin, pirarubicin, aclarubicin, epirubicin, oxaunomycin, and idarubicin. In the present embodiment, the anthracycline anticancer agent is preferably doxorubicin.

The present embodiment includes a step of measuring the expression level of GST-π contained in the biological sample containing breast cancer cells (hereinafter sometimes referred to as a "measuring step"). The measuring step may be performed by a known measurement method capable of measuring the expression level in protein or mRNA of GST-π and the method is not particularly limited. Examples of the known measurement method include an SDS polyacrylamide electrophoresis method, a two-dimensional electrophoresis method, an analysis method using a protein chip, an enzyme-linked immunosorbent assay method (ELISA), an immunofluorescence method, a Western blotting method, a dot blotting method, an immunoprecipitation method, an RT-PCR method, a Northern blotting method, an NASBA method, and a method using a DNA chip. In the present embodiment, a method for measuring the expression level in protein of GST-π is preferable as the measurement method.

The present embodiment includes a step of judging susceptibility of the breast cancer cells contained in the biological sample to an anthracycline anticancer agent as high when the expression level of GST-π obtained by the measuring process is high (hereinafter sometimes referred to as a "judging step").

In the present embodiment, the expression level of GST-π can be judged as high or low by the visual evaluation or quantification of the expression level of GST-π in accordance with the measurement method used in the measuring step. For example, in the measuring step, when the expression level of GST-π protein is measured by the Western blotting method, the expression level of GST-π can be judged as high or low by visually comparing a band of GST-π protein appeared on the membrane with a band of control. Further, the expression level of GST-π is quantified by scanning the band of GST-π protein appeared on the membrane with an image scanner and analyzing the signal strength. Alternatively, in the measuring step, when the expression level of GST-π protein is measured using a filter plate, the expression level of GST-π can be judged as high or low by visually comparing the fluorescence of GST-π protein appeared on the plate with the fluorescence of the control. Further, the expression level of GST-π is quantified by scanning the fluorescence of GST-π appeared on the plate with the image scanner and analyzing the fluorescence intensity. Then, the expression level of GST-π can also be judged as high or low by comparing the quantified expression level of GST-π with a predetermined threshold value (first threshold value). From the viewpoint of objectivity, it is preferable that the judging step in the present embodiment compares the quantified expression level of GST-π with the first threshold value and judges the expression level of GST-π as high or low.

Here, the first threshold value is a judging standard of the expression level of GST-π when judging the susceptibility of the breast cancer cells contained in the biological sample to the anthracycline anticancer agent. The first threshold value can be empirically set in accordance with the measurement method used in the measuring step. For example, the expression level of GST-π of a plurality of biological samples containing breast cancer cells with high susceptibility to the anthracycline anticancer agent is measured. Similarly, the expression level of a plurality of biological samples containing breast cancer cells with low susceptibility to the anthracycline anticancer agent is measured. On the basis of a plurality of expression levels of GST-π thus obtained, a value of the expression level of GST-π which can distinguish between biological samples containing breast cancer cells with high susceptibility to the anthracycline anticancer agent and biological samples containing breast cancer cells with low susceptibility to the anthracycline anticancer agent can be set as a first threshold value.

In the present embodiment, the measuring step can further measure an activity value of cyclin-dependent-kinase 1 (CDK1) contained in the biological sample containing breast cancer cells and an activity value of cyclin-dependent-kinase 2 (CDK2). When the expression level of GST-π obtained by the measuring step is high or when the value of CDK2 specific activity value/CDK1 specific activity value is higher than the second threshold value as a result of comparing a value of specific activity value of CDK2 obtained by the measuring step/specific activity value of CDK1 obtained by the measuring step (hereinafter sometimes referred to as CDK2 specific activity value/CDK1 specific activity value) with the second threshold value, the susceptibility of the breast cancer cells contained in the biological sample to the anthracycline anticancer agent can be judged as high. Thus, the susceptibility of the breast cancer cells to the anthracycline anticancer agent can be judged more accurately by using the value of CDK2 specific activity value/CDK1 specific activity value in addition to the expression level of GST-π.

Here, the specific activity value of CDK1 corresponds to a ratio of CDK1 exhibiting activities to CDKs1 present in the cells. The specific activity value of CDK1 can be calculated specifically from CDK1 activity value/CDK1 expression level. Here, the specific activity value of CDK2 corresponds to a ratio of CDK2 exhibiting activities to CDKs2 present in the cells. The specific activity value of CDK2 can be calculated specifically from CDK2 activity value/CDK2 expression level.

CDK1 and CDK2 activity values are kinase activity levels calculated, for example, from the amount of a substrate to be phosphorylated by CDK (the unit is represented by U (unit)). An example of the substrate to be phosphorylated by CDK1 and CDK2 includes histone H1.

The CDK1 and CDK2 activity values can be measured by a known method for measuring CDK activity. Examples of the known method for measuring CDK activity include a method for measuring the activity using a radioactive label and a method for measuring the activity using no radioactive label. As the method for measuring the activity using a radioactive label, for example, a method which includes preparing a sample from a biological sample containing cancer cells, allowing 32P to be incorporated into a substrate protein using the sample and 32P-labeled ATP ([γ-32P] ATP), measuring the amount of the label in the 32P-labeled phosphorylated substrate, and quantitatively determining the activity value of CDK1 or the activity value of CDK2 on the basis of a calibration curve prepared using standard samples is listed. As the method for measuring the activity without using no radioactive label, for example, a method described in U.S. Patent Application Publication No. 2002/164673 is listed. The method described in U.S. Patent Application Publication No. 2002/164673 is a method which includes preparing a sample from a biological sample containing cancer cells, reacting the substrate protein in the sample with adenosine-5'-O-(3-thiotriphosphate) (ATPγS) to introduce a monothiophosphate group into a serine or threonine residue of the substrate protein, binding a fluorescently-labeled substance or a labeled enzyme to a sulfur atom in the introduced monothiophosphate group thereby labeling the substrate protein, measuring the amount of the labeled thiophosphorylated substrate (or the amount of the fluorescent substance in the case where the fluorescent substance is used), and quantitatively determining the activity level based on a calibration curve previously prepared using standard samples.

The sample to be used for measuring the activity is prepared by specifically collecting CDK1 or CDK2 from the biological sample containing cancer cells being measured. In this case, the sample is prepared by using an anti-CDK antibody specific to either CDK1 or CDK2. In each case, CDK1 other than activated CDK1 or CDK2 other than activated CDK2 are included. For example, in the case of CDK1, a complex having the CDK1 inhibitor bound to a cyclin/CDK1 complex is included. When an anti-CDK1 antibody is used, the single CDK1, a complex of CDK1 with cyclin and/or CDK1 inhibitor, a complex of CDK1 with another compound and the like are included. Therefore, the CDK1 activity value is measured as a unit (U) of the phosphorylated substrate under a condition where active and inactive CDKs1 and a wide variety of competitive reactions occur. In the case of CDK2, the measurement is performed under the same condition as the case of CDK1.

The expression level of CDK1 or the expression level of CDK2 is the CDK1 amount or the CDK2 amount (a unit corresponding to the molecule number) contained in a solubilized liquid of the biological sample containing cancer cells to be measured, and can be measured by a conventionally known method for measuring a mass of proteins from a protein mixture. For example, the ELISA method and the Western blot method may be used or a method described in Japanese Patent Application Laid-Open (JP-A) No. 2003-130971 can be used for the measurement. CDK1 and CDK2 may be captured using a specific antibody. For example, in the case of CDK1, all the CDKs1 present in the cells (containing a CDK1 carrier, a complex of CDK1 with cyclin and/or CDK1 inhibitor, and complexes of CDK1 and other compounds) can be captured by using an anti-CDK1 antibody. In the case of CDK2, the expression level of CDK2 can be measured in the same procedure as the case of CKD1.

Here, the second threshold value is a judging standard of the value of CDK2 specific activity value/CDK1 specific activity value when judging the susceptibility of the breast cancer cells contained in the biological sample to the anthracycline anticancer agent. The threshold value can be empirically set in accordance with the measurement method used in the measuring step. For example, the value of CDK2 specific activity value/CDK1 specific activity value of a plurality of biological samples containing breast cancer cells with high susceptibility to the anthracycline anticancer agent is measured. Similarly, the value of CDK2 specific activity value/CDK1 specific activity value of a plurality of biological samples containing breast cancer cells with low susceptibility to the anthracycline anticancer agent is measured. On the basis of a plurality of values of CDK2 specific activity value/CDK1 specific activity value thus obtained, a value of CDK2 specific activity value/CDK1 specific activity value which can distinguish between biological samples containing breast cancer cells with high susceptibility to the anthracycline anticancer agent and biological samples containing breast cancer cells with low susceptibility to the anthracycline anticancer agent can be set as a threshold value.

In the present embodiment, it may be configured that the judging step judges the susceptibility of the breast cancer cells contained in the biological sample to the anthracycline anticancer agent as low when the expression level of GST-π obtained by the measuring step is low and the value of CDK2 specific activity value/CDK1 specific activity value is lower than the threshold value as a result of comparing the value of CDK2 specific activity value/CDK1 specific activity value with the second threshold value. Thus, the susceptibility of the breast cancer cells to the anthracycline anticancer agent can be judged as low by using the expression level of GST-π and the value of CDK2 specific activity value/CDK1 specific activity value. As a result, an unnecessary administration of the anthracycline anticancer agent to the patient can be avoided.

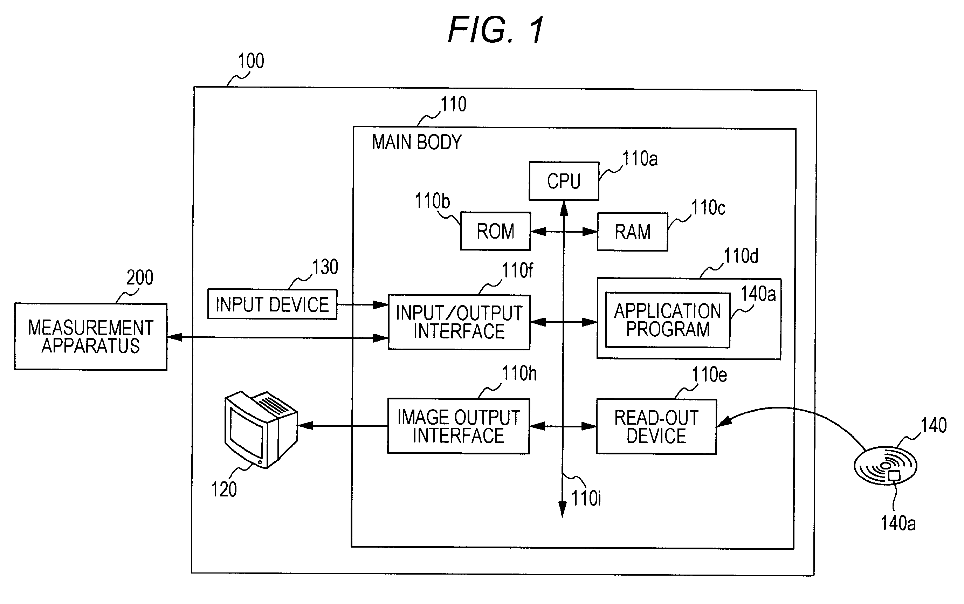

In the present embodiment, the computer program can make the computer execute the judgment process of the susceptibility of the breast cancer cells contained in the biological sample to the anthracycline anticancer agent. An example of a specific computer is shown in Fig. 1.

A computer 100 is mainly configured by a main body 110, a display unit 120, and an input device 130. In the main body 110, a CPU110a, a ROM 110b, a RAM 110c, a hard disk 110d, a read-out device 110e, and an input/output interface 110f, and an image output interface 110h are data-communicably connected by a bus 110i.

The CPU 110a can execute computer programs stored in the ROM 1101 b and the computer programs loaded in the RAM 110c.

The ROM 110b is configured by mask ROM, PROM, EPROM, EEPROM, and the like, and is recorded with computer programs to be executed by the CPU 110a, and data used for the same.

The RAM 110c is configured by SRAM, DRAM, and the like. The RAM 110c is used to read out the computer programs recorded on the ROM 110b and the hard disc 110d. In executing the computer program, the RAM 110c is used as a work region of the CPU 110a.

The hard disc 110d is installed with various computer programs to be executed by the CPU 110a such as operating system and application system program, as well as data used in executing the computer program. The application program 140a to be described later is also installed in the hard disc 110d.

The read-out device 110e is configured by flexible disc drive, CD-ROM drive, DVD-ROM drive, and the like, and is able to read out computer programs and data recorded on a portable recording medium 140. The application program 140a according to the judgment of the computer is stored in the portable recording medium 140, so that the CPU 110a can read out the application program 140a from the portable recording medium 140 and install the application program 140a to the hard disk 110d.

Operating system providing graphical user interface environment such as Windows (registered trademark) manufactured and sold by US Microsoft Corporation is installed in the hard disc 110d. In the following description, the application program 140a according to the above-described judgment is assumed to be operating on the operating system.

The input/output interface 110f includes a serial interface such as USB, IEEE1394, and RS-232C; a parallel interface such as SCSI, IDE, and IEEE1284; and an analog interface such as D/A converter and A/D converter. The input/output interface 110f is connected to the input device 130 including a keyboard and a mouse, and users can use the input device 130 to input data into the main body 110 of the computer.

A measurement apparatus 200 is connected to the input/output interface 110f. Thus, the main body 110 of the computer can acquire the expression levels of GST-π from the measurement apparatus 200 via the input/output interface 110f.

The image output interface 110h is connected to the display unit 120 configured by LCD, CRT, or the like, and is configured to output an image signal corresponding to the image data provided from the CPU 110a to the display unit 120. The display unit 120 outputs image data according to the input image signal. The display unit 120 outputs the judged result provided from the CPU 110a to be described later.

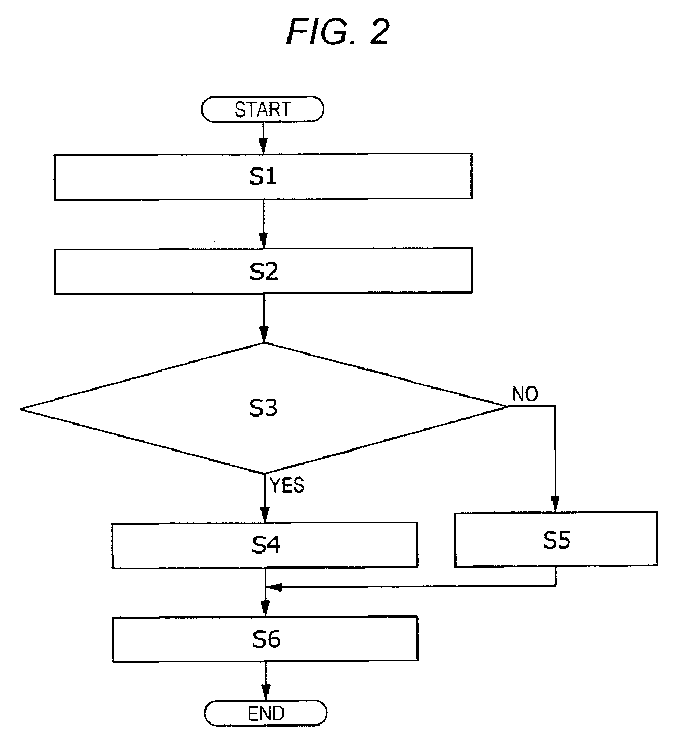

Fig. 2 is a flow chart showing operation of the application program 140a to execute the judgment process in accordance with the expression levels of GST-π of the breast cancer cells contained in the biological sample. First, the data related to the expression levels of GST-π of the breast cancer cells contained in the biological sample which has been acquired by the measurement apparatus 200 is input into the main body 110 of the computer via the input/output interface 110f. The CPU 110a calculates the expression levels of GST-π based on the data related to the expression levels of GST-π and stores it in the RAM 110c (step S1). As the data related to the expression levels of GST-π of the breast cancer cells contained in the biological sample, the fluorescence intensity measured with a fluorescence intensity measurement apparatus (Plate Reader (Infinite F200, manufactured by Tecan Trading AG)) in Example 1 to be described later is input to the main body 110 of the computer.

The CPU 110a reads out a threshold value which has been stored in a memory 110d as data of the application program 140a in advance and executes the comparison of the threshold value and the expression level of GST-π (step S2). Here, the threshold value is a threshold value 3 of the expression level of GST-π in Example 1 to be described later.

Then, the CPU 110a judges whether the expression level of GST-π is the threshold value or more (step S3).

When the expression level of GST-π is the threshold value or more, the CPU 110a judges the susceptibility of the breast cancer cells contained in the biological sample to the anthracycline anticancer agent as "high" (step S4). In contrast, when the expression level of GST-π is less than the threshold value, the CPU 110a makes the judgment process of the susceptibility of the breast cancer cells contained in the biological sample to the anthracycline anticancer agent "pending" (step S5). In this case, 9 of the 29 types of breast cancer cell lines in Example 1 to be described later are judged to have a "high" susceptibility to the anthracycline anticancer agent and 20 types of breast cancer cell lines are judged as "pending".

The CPU 110a stores the judged results in the RAM 110c and outputs them on the display unit 120 via the image output interface 110h (step S6).

In the present embodiment, the expression levels of GST-π are acquired from the measurement apparatus 200 via the input/output interface 110f, however the present invention is not limited thereto. For example, it may be configured that the expression levels of GST-π are acquired from the input device 130 after an operator's input operation.

In addition to the expression levels of GST-π, the computer program in the present embodiment can make the computer perform the judgment process of the susceptibility of breast cancer cells to an anthracycline anticancer agent using the value of CDK2 specific activity value/CDK1 specific activity value.

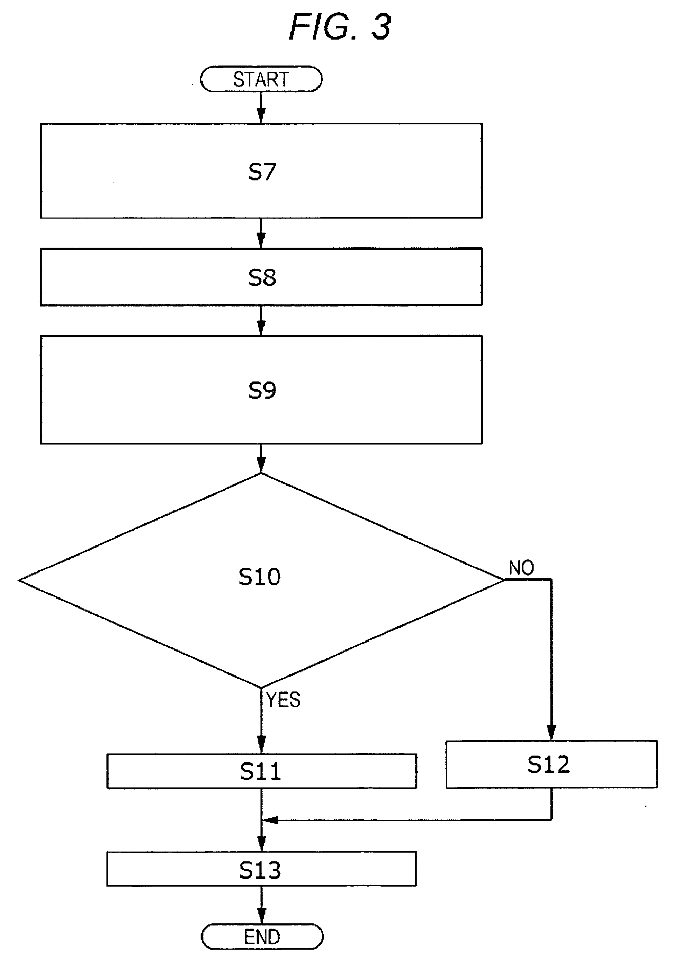

Fig. 3 is a flow chart showing the operation of the application program 140a which judges the susceptibility of breast cancer cells to an anthracycline anticancer agent as high in accordance with the expression levels of GST-π and CDK1 and CDK2 specific activity values. First, the data related to the expression levels of GST-π of the breast cancer cells contained in the biological sample, the activity value and expression level of CDK1, and the activity value and expression level of CDK2 is input to the main body 110 of the computer via the input/output interface 110f. The CPU 110a calculates the expression levels of GST-π, the activity value and expression level of CDK1, and the activity value and expression level of CDK2 based on the data related to the expression levels of GST-π, the activity value and expression level of CDK1, and the activity value and expression level of CDK2 and respectively stores them in the RAM 110c (step S7). As the data related to the expression levels of GST-π of the breast cancer cells contained in the biological sample, the activity value and expression level of CDK1, and the activity value and expression level of CDK2, the fluorescence intensity measured with a fluorescence intensity measurement apparatus (Plate Reader (Infinite F200, manufactured by Tecan Trading AG)) in Example 3 to be described later is input to the main body 110 of the computer.

The CPU 110a calculates the value of CDK2 specific activity value/CDK1 specific activity value from the activity value and expression level of CDK1 and the activity value and expression level of CDK2 which are stored in the RAM 110c (step S8).

The CPU 110a reads out the first and second threshold values which has been stored in the memory 110d as data of the application program 140a in advance and executes the comparison of the first threshold value and the expression level of GST-π and the comparison of the second threshold value and the value of CDK2 specific activity value/CDK1 specific activity value (step S9). Here, the first threshold value is a threshold value 3 of the expression level of GST-π in Example 3 to be described later. The second threshold value is a threshold value of 7.41 of the value of CDK2 specific activity value/CDK1 specific activity value in Example 3 to be described later.

Then, the CPU 110a judges whether the expression level of GST-π is the first threshold value or more or the value of CDK2 specific activity value/CDK1 specific activity value is the second threshold value or more (step S10).

When the expression level of GST-π is the first threshold value or more or when the value of CDK2 specific activity value/CDK1 specific activity value is the second threshold value or more, the CPU 110a judges the susceptibility of the breast cancer cells contained in the biological sample to the anthracycline anticancer agent as "high" (step S11). In contrast, when the expression level of GST-π is less than the first threshold value or the value of CDK2 specific activity value/CDK1 specific activity value is less than the second threshold value, the CPU 110a makes the judgment process of the susceptibility of the breast cancer cells contained in the biological sample to the anthracycline anticancer agent "pending" (step S12). In this case, 16 of the 27 types of breast cancer cell lines in Example 3 to be described later are judged to have a "high" susceptibility to the anthracycline anticancer agent and 11 types of breast cancer cell lines are judged as "pending".

The CPU 110a stores the judged results in the RAM 110c and outputs them on the display unit 120 via the image output interface 110h (step S13).

In addition to the expression levels of GST-π, the computer program in the present embodiment can judge whether breast cancer cells have a "low" susceptibility to the anthracycline anticancer agent using the value of CDK2 specific activity value/CDK1 specific activity value.

Fig. 4 is a flow chart showing the operation of the application program 140a which judges the susceptibility of breast cancer cells to an anthracycline anticancer agent as high or low in accordance with the expression levels of GST-π and CDK1 and CDK2 specific activity values. First, the data related to the expression levels of GST-π of the breast cancer cells contained in the biological sample acquired from measurement apparatus 200, the activity value and expression level of CDK1, and the activity value and expression level of CDK2 is input to the main body 110 of the computer via the input/output interface 110f. The CPU 110a calculates the expression levels of GST-π, the activity value and expression level of CDK1, and the activity value and expression level of CDK2 based on the data related to the expression levels of GST-π, the activity value and expression level of CDK1, and the activity value and expression level of CDK2 and respectively stores them in the RAM 110c (step S14). As the data related to the expression levels of GST-π of the breast cancer cells contained in the biological sample, the activity value and expression level of CDK1, and the activity value and expression level of CDK2, the fluorescence intensity measured with a fluorescence intensity measurement apparatus (Plate Reader (Infinite F200, manufactured by Tecan Trading AG)) in Example 3 to be described later is input to the main body 110 of the computer.

The CPU 110a calculates the value of CDK2 specific activity value/CDK1 specific activity value from the activity value and expression level of CDK1 and the activity value and expression level of CDK2 which are stored in the RAM 110c (step S15).

The CPU 110a reads out the first and second threshold values which has been stored in the memory 110d as data of the application program 140a in advance and executes the comparison of the first threshold value and the expression level of GST-π and the comparison of the second threshold value and the value of CDK2 specific activity value/CDK1 specific activity value (step S16). Here, the first threshold value is a threshold value 3 of the expression level of GST-π in Example 3 to be described later. The second threshold value is a threshold value of 7.41 of the value of CDK2 specific activity value/CDK1 specific activity value in Example 3 to be described later.

Then, the CPU 110a judges whether the expression level of GST-π is less than the first threshold value or the value of CDK2 specific activity value/CDK1 specific activity value is less than the second threshold value (step S17).

When the expression level of GST-π is less than the first threshold value or the value of CDK2 specific activity value/CDK1 specific activity value is less than the second threshold value, the CPU 110a judges the susceptibility of the breast cancer cells contained in the biological sample to the anthracycline anticancer agent as "low" (step S18). In contrast, when the expression level of GST-π is the first threshold value or more or when the value of CDK2 specific activity value/CDK1 specific activity value is the second threshold value or more, the CPU 110a judges the susceptibility of the breast cancer cells contained in the biological sample to the anthracycline anticancer agent as "high" (step S19). In this case, 7 of the 27 types of breast cancer cell lines in Example 3 to be described later are judged to have a "low" susceptibility to the anthracycline anticancer agent and 20 types of breast cancer cell lines are judged to have a "high" susceptibility to the anthracycline anticancer agent.

The CPU 110a stores any of the judged results in the RAM 110c and outputs them on the display unit 120 via the image output interface 110h (step S20).

In the present embodiment, the expression levels of GST-π, the activity value and expression level of CDK1, and the activity value and expression level of CDK2 are acquired from the measurement apparatus 200 via the input/output interface 110f, however the present invention is not limited thereto. For example, it may be configured that the expression level of GST-π is acquired from the input device 130 after an operator's input operation.

In the present embodiment, the expression levels of GST-π are acquired from the measurement apparatus 200 via the input/output interface 110f, however the present invention is not limited thereto. For example, it may be configured that the expression levels of GST-π are acquired from the input device 130 after an operator's input operation.

### Examples

In the following examples, the present invention will be explained in more details, however it is not intended that the present invention is limited to the following embodiments.

### (Reference example 1)

### <Culture of breast cancer cell line>

Human breast cancer cell lines such as AU565 (ATCC CRL-2351), BT-20 (ATCC HTB-19), BT-474 (ATCC HTB-20), BT-549 (ATCC HTB-122), HCC1569 (ATCC CRL-2330), HCC1937 (ATCC CRL-2336), Hs578T (ATCC HTB-126), SK-BR-3 (ATCC HTB-30), ZR-75-1 (ATCC CRL-1500), ZR-75-30 (ATCC CRL-1504), HCC1419 (ATCC CRL-2326), HCC202 (ATCC CRL-2316), BT-483 (ATCC HTB-121), CAMA-1 (ATCC HTB-21), HCC1954 (ATCC CRL-2338), MCF7 (ATCC HTB-22), T-47D (ATCC HTB-133), HCC1500 (ATCC CRL-2329), HCC1428 (ATCC CRL-2327), HCC1806 (ATCC CRL-2335), MDA-MB-415 (ATCC HTB-128), MDA-MB-231 (ATCC HTB-26), MDA-MB-361 (ATCC HTB-27), MDA-MB-435S (ATCC HTB-129), MDA-MB-453 (ATCC HTB-131), UACC-812 (ATCC CRL-1897), MDA-MB-157 (ATCC HTB-24), MDA-MB-436 (ATCC HTB-130), UACC-893 cells (ATCC CRL-1902) were purchased from American Type Culture Collection(ATCC) and the cells were cultured under ATCC-recommended culture conditions.

The ATCC-recommended culture conditions for each cell line such as basal media, percent concentrations of fetal bovine serum (FBS) in the basal media, additives for the culture media, and culturing environments are shown in Table 1.

**[Table 1]**

| Cell names | Basal media | FBS (%) | Additives | Culturing environments |
|---|---|---|---|---|
| AU565 | RPMI 1640 | 10 | | 37°C, 5% CO2 |
| BT-20 | MEM | 10 | | 37°C, 5% CO2 |
| BT-474 | DMEM | 10 | | 37°C, 5% CO2 |
| BT-483 | RPMI 1640 | 20 | 10 ug/ ml bovine insulin | 37°C, 5% CO2 |
| BT-549 | RPMI 1640 | 10 | 0.0231 U/ ml (1 ug/ ml) bovine insulin | 37°C, 5% CO2 |
| CAMA-1 | MEM | 10 | | 37°C, 5% CO2 |
| HCC202 | RPMI 1640 | 10 | | 37°C, 5% CO2 |
| HCC1419 | RPMI 1640 | 10 | | 37°C, 5% CO2 |
| HCC1428 | RPMI 1640 | 10 | | 37°C, 5% CO2 |
| HCC1500 | RPMI 1640 | 10 | | 37°C, 5% CO2 |
| HCC1569 | RPMI 1640 | 10 | | 37°C, 5% CO2 |
| HCC1806 | RPMI 1640 | 10 | | 37°C, 5% CO2 |
| HCC1937 | RPMI 1640 | 10 | | 37°C, 5% CO2 |
| HCC1954 | RPMI 1640 | 10 | | 37°C, 5% CO2 |
| Hs578T | DMEM | 10 | 10 ug/ ml bovine insulin | 37°C, 5% CO2 |
| MCF7 | MEM | 10 | 10 ug/ ml bovine insulin | 37°C, 5% CO2 |
| MDA-MB-157 | Leibovitz's L-15 | 10 | 2 mM L- glutamine | 37°C, 100% air |
| MDA-MB-231 | Leibovitz's L-15 | 10 | 2 mM L- glutamine | 37°C, 100% air |
| MDA-MB-361 | Leibovitz's L-15 | 20 | 2 mM L- glutamine | 37°C, 100% air |
| MDA-MB-415 | Leibovitz's L-15 | 15 | 10 ug/ ml bovine insulin, 10 ug/ml gluthione, 2 mM L-glutamine | 37°C, 100% air |
| MDA-MB-435S | Leibovitz's L-15 | 10 | 10 ug/ ml bovine insulin, 2 mM L- glutamine | 37°C, 100% air |
| MDA-MB-436 | Leibovitz's L-15 | 10 | 10 ug/ ml bovine insulin, 10 ug/ml gluthione, 2 mM L-glutamine | 37°C, 100% air |
| MDA-MB-453 | Leibovitz's L-15 | 10 | 2 mM L- glutamine | 37°C, 100% air |
| SK-BR-3 | McCoy's 5a | 10 | 2 mM L- glutamine | 37°C, 5% CO2 |
| T-47D | RPMI 1640 | 10 | 0.2 units/ ml (10 ug/ ml) bovine insulin | 37°C, 5% CO2 |
| UACC-81 2 | Leibovitz's L-15 | 20 | 20 ng/ml human EGF, 2 mM L- glutamine | 37°C, 100% air |
| UACC-89 3 | Leibovitz's L-15 | 10 | 2 mM L- glutamine | 37°C, 100% air |
| ZR-75-1 | RPMI 1640 | 10 | | 37°C, 5% CO2 |
| ZR-75-30 | RPMI 1640 | 10 | | 37°C, 5% CO2 |

### (Reference example 2)

### <Production of test samples>

29 types of breast cancer cell lines shown in Reference example 1 respectively were inoculated into a culture medium in a 15-cm dish under the culture conditions of Reference example 1 and cultured at a confluence of 70 to 80%. After the culture, the culture medium was removed from the 15-cm dish and 10 mL of phosphate buffered saline was placed in the 15-cm dish. Then, the cells in the 15-cm dish were washed with the phosphate buffered saline. Subsequently, 3 mL of a trypsin EDTA solution (0.05% trypsin, 0.53 mM EDTA) was added to the cells of the 15-cm dish, which was incubated at 37°C for 5 to 10 minutes and the cells were removed from a flask. 10 mL or more of the culture medium was added to the flask to stop the reaction with trypsin and the cells were recovered.

The recovered cells were respectively transferred into microtubes. Then, a solubilizing reagent (50 mM Tris-HCl (pH 7.5)), 5 mM EDTA (pH 8.0), 50 mM NaF, 1 mM Na3VO4, 0.1% NP40, 0.2% protease inhibitor cocktail (manufactured by Sigma-Aldrich Corporation) was dispensed into each microtube in an amount of 150 to 250 µL so that the final concentration of proteins in the solution was 2 ug/uL. Subsequently, each microtube was placed in a homogenizer, followed by homogenization and centrifugation at 4°C or less at 15000 rpm for 5 minutes using a micro high-speed centrifuge (CF15RX, manufactured by Hitachi, Ltd.). The supernatant of each microtube was transferred into a 1.5 mL microtube left on ice and used as a test sample.

### (Example 1)

### <Judgment of susceptibility of breast cancer cells to anthracycline anticancer agent based on expression levels of GST-π>

The Judgment of the susceptibility of breast cancer cells to an anthracycline anticancer agent based on the expression levels of GST-π of breast cancer cells contained in the biological sample was examined in the following manner.

### <Measurement of IC50>

29 types of breast cancer cell lines shown in Reference example 1 were inoculated into wells on two sheets of microplates at 5000 cells/100uL/well using culture media containing doxorubicin at 0nM, 250nM, 500nM, 750nM, 1000nM, and 2000nM concentrations, followed by culturing for 24 hours.

Culture media containing doxorubicin at respective concentrations were prepared and the culture medium exchange was performed. Immediately after the culture-medium exchange, the growth of the cells on one of the microplates was measured using Celltiter Glo Luminescent Cell Viavility Assay (Cat#G7571: manufactured by Promega KK.). The remaining microplate was cultured under the culture conditions shown in Reference example 1 for 72 hours after the culture-medium exchange. Thereafter, the growth of the cells was measured using Celltiter Glo Luminescent Cell Viavility Assay (Cat#G7571: manufactured by Promega KK.).

On the basis of the number of the living cells measured, the concentration (IC50) of doxorubicin (the number of living cells in the culture medium without treatment with doxorubicin/the number of living cells in the culture medium obtained by treatment with doxorubicin x 100 = 50) was determined.

Here, 392 nM (median of IC50) was defined as a threshold value, breast cancer cell lines having a value more than the threshold value were defined as nonsensitive cell lines, and breast cancer cell lines having a value less than the threshold value were defined as sensitive cell lines.

### <Measurement of expression levels of GST-π>

The reagents to be used were prepared in the following manner.

The reagents used for measurement of the expression levels of GST-π are shown below.

### (1) Blocking reagent

Block Ace powder (manufactured by Dainippon Sumitomo Pharma Co., Ltd.) was dissolved in ultrapure water so as to have a final concentration of 4% to prepare a blocking reagent.

### (2) Primary antibody reagent

GST-π antibodies (manufactured by BD Transduction Laboratories) were dissolved in the blocking solution diluted 10-fold so as to be 5 ug/mL to prepare a primary antibody reagent.

### (3) Tris-buffered saline (TBS)

A 10 x TBS solution (2-Amino-2-hydroxymethyl-1, 3-propanediol: 30.28 g, NaCl: 87.7g, 6N HCl: 110 g/L) was diluted 10-fold to prepare a TBS solution.

### (4) Secondary antibody reagent

Bovine serum albumin (BSA) was added to the TBS solution, which was dissolved in ultrapure water so as to have a final concentration of 1% and a TBS (1% BSA) solution was prepared. Goat Anti-Mouse IgGs (H+L) -BIOT Human/Mouse (manufactured by SouthernBiotech) were dissolved in the obtained TBS (1% BSA) solution so as to be 30 ug/mL and a secondary antibody reagent was prepared.

### (5) Fluorescent labeling reagent

Fluorescein StreptAvidin (manufactured by Vector Laboratories) was dissolved in the TBS (1% BSA) solution so as to be 10 µg/mL to prepare a fluorescent labeling reagent.

The expression levels of GST-π were measured in the following manner. Each test sample of the 29 types of breast cancer cell lines shown in Reference example 1 which had been prepared in the manner described in Reference example 2 was dispensed into filter plates (Multi Screen HTSPSQ Plates) at 10 µg/well and the water content was removed by aspiration. Thereafter, the blocking reagent was dispensed into each well at 100 µL/well and it was aspirated.

After the aspiration, the primary antibody reagent was dispensed into each well at 50 µL/well and each of the reagents was immediately aspirated. Then, the primary antibody reagent was again dispensed into each well at 50 uL/well and left at rest for 2 hours, followed by removal of the primary antibody by aspiration. Subsequently, a washing process for dispensing the TBS solution into each well at 300 µL/well and aspirating each of the solutions was performed 4 times. After the washing, the secondary antibody reagent was dispensed into each well at 50 µL/well and each of the reagents was immediately aspirated. Then, the secondary antibody reagent was again dispensed into each well at 50 uL/well and left at rest for 1 hour, followed by removal of the secondary antibody by aspiration. Subsequently, the washing process for dispensing the TBS solution into each well at 300 µL/well and aspirating each of the solutions was performed twice. After the washing, the fluorescent labeling reagent was dispensed into each well at 100 µL/well and then each of the reagents was immediately aspirated. Subsequently, the washing process for dispensing the TBS solution into each well at 300 µL/well and aspirating each of the solutions was performed 4 times, followed by drying of the filter plates.

After the drying, the fluorescence intensity was measured with the Plate Reader (Infinite F200, manufactured by Tecan Trading AG). The expression levels of GST-π for each test sample were calculated using a calibration curve prepared by using GSTP1 recombinant Proteins (Cat No.H00002950-P01, manufactured by Abnova Corporation.) as calibrators, placing them on a filter plate at each concentration (0 ng/100 uL/well, 10 ng/100 uL/well, 30 ng/100 uL/well, 50 ng/100 uL/well) and measuring the fluorescence intensity by Infinite F200 (manufactured by Tecan Trading AG).

Here, the threshold value of the expression level of GST-π was defined as 3, breast cancer cell lines having a value more than the threshold value were defined as cell lines with a high expression level of GST-π, and breast cancer cell lines having a value less than the threshold value were defined as cell lines with a low expression level of GST-π.

The results of this example are shown in Fig. 5.

From the results shown in Fig. 5, among 29 types of breast cancer cell lines, cell lines with a high expression level of GST-π tend to have a high susceptibility to doxorubicin. As a result, it is shown that when the expression level of GST-π of breast cancer cells is high, the susceptibility of breast cancer cells to an anthracycline anticancer agent can be judged as high.

### (Example 2)

### <Expression levels of GST-π in recurring specimen and non-recurring specimen>

On the basis of expression levels of GST-π in 9 specimens of breast cancer patients before receiving chemotherapy including the anthracycline anticancer agent, the tendency of expression levels of GST-π in recurring specimens and non-recurring specimens given chemotherapy after surgery including the anthracycline anticancer agent was examined.

The 9 specimens were tumor tissues containing breast cancer removed by surgery. They had been kept frozen at -80°C until the measurement. Among the 9 specimens, 5 specimens were recurring specimens and 4 specimens were non-recurring specimens.

Disease-free recurrence periods in the recurring specimens were 210, 318, 566, 761, and 1377 days, while the disease-free recurrence periods in the non-recurring specimens were 1594, 1599, 1835, and 2024 days.

### <Preparation of test samples>

9 specimens were subjected to solubilization treatment as follows and used as test samples.

Respective specimens were cut into about 4- or 3-mm squares on a petri dish placed on dry ice and transferred into microtubes.

The solubilizing reagent (50 mM Tris-HCl (pH 7.5)), 5 mM EDTA (pH 8.0), 50 mM NaF, 1 mM Na3VO4, 0.1% NP40, 0.2% protease inhibitor cocktail (manufactured by Sigma-Aldrich Corporation) was dispensed into each microtube in an amount of 400 µL for a 4 mm square specimen or in an amount of 300 µL for a 3 mm square specimen. Subsequently, each sample tube was placed in the homogenizer, followed by homogenization and centrifugation at 4(C or less at 15000 rpm for 5 minutes using the micro high-speed centrifuge (CF15RX, manufactured by Hitachi, Ltd.). Then, the supernatant of each sample tube was transferred into a 1.5 mL microtube left on ice and used as a test sample.

### <Measurement of expression levels of GST-π>

The test samples of 9 specimens prepared by the above-described method were measured by the method described in Example 1.

The results of this example are shown in Fig. 6. Fig. 6 shows a boxplot.

From the results shown in Fig. 6, it is shown that, among 9 specimens, the recurring specimen has a low expression level of GST-π and the non-recurring specimen has a high expression level of GST-π. As a result, it is suggested that the tendency corresponds to the result that cell lines with a high expression level of GST-π have a high susceptibility to the anthracycline anticancer agent in Example 1.

### (Example 3)

### <Judgment of susceptibility of breast cancer cells to anthracycline anticancer agent based on expression levels of GST-π and value of CDK2 specific activity value/CDK1 specific activity value>

The judgment of the susceptibility of breast cancer cells to an anthracycline anticancer agent based on the combination of the expression levels of GST-π of breast cancer cells contained in the biological sample with the value of CDK2 specific activity value/CDK1 specific activity value was examined.

### <Measurement of IC50>

29 types of breast cancer cell lines shown in Reference example 1 were measured by the method described in Example 1. Here, 392 nM (median of IC50) was defined as a threshold value, breast cancer cell lines having a value more than the threshold value were defined as nonsensitive cell lines, and breast cancer cell lines having a value less than the threshold value were defined as sensitive cell lines.

### <Measurement of expression levels of GST-π>

29 types of breast cancer cell lines shown in Reference example 1 were measured by the method described in Example 1. Here, the threshold value of the expression level of GST-π was defined as 3, breast cancer cell lines having a value more than the threshold value were defined as cell lines with a high expression level of GST-π, and breast cancer cell lines having a value less than the threshold value were defined as cell lines with a low expression level of GST-π.

### <Measurement of CDK1 and CDK2 activity values>

The reagents to be used were prepared in the following manner.
(1) Membrane wash solution

| | |
|---|---|
| 10xTBS solution | 100 mL |
| Ultrapure water | 900 mL |

(2) Immunoprecipitation buffer

| | |
|---|---|
| 1 M Tris-HCl(pH7.4) | 10.0mL |
| 10% NP-40 Alternative PROTEIN GRADE (manufactured by CALBIOCHEM) | 2.0 mL |
| 10% sodium azide solution | 1.9 mL |
| Ultrapure water | QS-200 mL |

(3) CDK antibody diluent

| | |
|---|---|
| Sucrose (manufactured by KISHIDA CHEMICAL Co., Ltd.) | 17.1 g |
| 1 M Tris-HCl (pH7.4) | 1.25 mL |
| 3M NaCl solution | 2.5 mL |
| 10% sodium azide solution | 470 (L |
| Ultrapure water | QS-50 mL |

(4) Anti-CDK1 antibody reagent

| | |
|---|---|
| CDK antibody diluent | 1.9 mL |
| Anti-CDK1 antibody (448 (g/mL) | 17.5 mL |
| 10% sodium azide solution | 190 (L |
| Immunoprecipitation buffer | 840 (L |

(5) Anti-CDK2 antibody reagent

| | |
|---|---|
| CDK antibody diluents | 1.6 mL |
| Anti-CDK2 antibody (360 µg/mL) | 8.3 mL |
| 10% sodium azide solution | 100 (L |
| Immunoprecipitation buffer | 960 (L |

(6) Antibody reagent for back ground

| | |
|---|---|
| CDK antibody diluents | 9.5 mL |
| Rabbit IgG (manufactured by CALBIOCHEM)(12.5 mg/mL) | 440 (L |
| Immunoprecipitation buffer | 960 (L |

(7) Immunoprecipitation wash solution 1

| | |
|---|---|
| 1 M Tris-HCl (pH7.4) | 25.0 mL |
| 10% NP-40 Alternative PROTEIN GRADE (manufactured by CALBIOCHEM) | 50.0 mL |
| 10% sodium azide solution | 4.7 mL |
| Ultrapure water | QS-500 mL |

(8) Immunoprecipitation wash solution 2

| | |
|---|---|
| 1 M Tris-HCl (pH7.4) | 12.5 mL |
| 3M NaCl solution | 25.0 mL |
| 10% sodium azide solution | 2.2 mL |
| Ultrapure water | QS-250 mL |

(9) Immunoprecipitation wash solution 3

| | |
|---|---|
| 1 M Tris-HCl (pH7.4) | 12.5 mL |
| 10% sodium azide solution | 2.3 mL |
| Ultrapure water | QS-250 mL |

(10) Kinase reaction reagent

| | |
|---|---|
| Ultrapure water | 17.9 mL |
| 1 M Tris-HCl (pH7.4) | 1.22 mL |
| 10% sodium azide solution | 200 (L |
| 1 M magnesium chloride solution | 450 (L |
| 25 mM ATP-(S solution | 1.8 mL |
| 5 mg/mL Histone H1 solution | 900 (L |

(11) Fluorescent labeling reaction buffer

| | |
|---|---|
| 2.2M MOPS-NaOH (pH7.4) | 34.1 mL |
| 0.5M EDTA (pH8.0) | 2.5 mL |
| 10% sodium azide solution | 2.1 mL |
| Ultrapure water | QS-250 mL |

(12) Fluorescent labeling reagent

| | |
|---|---|
| 5-(Iodoacetamido)fluorescein (manufactured by Molecular Probes) | 25 mg |
| DMSO | 6 mL |
| Fluorescent labeling reaction buffer | 116 mL |

(13) Fluorescent labeling reaction quenching solution

| | |
|---|---|
| 2.2M MOPS-NaOH (pH7.4) | 455 mL |
| N-Acetyl-L-Cysteine (manufactured by Nacalai Tesque, Inc.) | 2.45 g |
| 10% sodium azide solution | 450 µL |
| Ultrapure water | 43.5 mL |

(14) Fluorescence enhancing reagent

| | |
|---|---|
| Blocking One (manufactured by Nacalai Tesque, Inc.) | 100 mL |
| 10% sodium azide solution | 10 mL |
| Ultrapure water | 890 mL |

The activity values of CDK1 and CDK2 were measured in the following manner.

20% Beads Sol (Protein A bead: 300 µL, immunoprecipitation buffer: 900 µL) was dispensed into each well of a filter plate (manufactured by Millipore Corporation) at 30 µL/well. Additionally, an anti-CDK1 antibody reagent, an anti-CDK2 antibody reagent, and an antibody reagent for back ground were respectively dispensed into corresponding wells in an amount of 90 µL. Each test sample of the types of breast cancer cell lines shown in Reference example 1 which had been prepared in the manner described in Reference example 2 was dispensed into each well at 30 µL/well, and stirred at 4(C for 2 hours using a Plate Shaker (SHK-10, manufactured by Masuda Corporation) to react CDK1 with an anti-CDK1 antibody and react CDK2 with an anti-CDK2 antibody.

After the reaction, a washing process was performed in the following manner. The reagent in each well was aspirated, the immunoprecipitation wash solution 1 was dispensed into each well at 200 (L/well, followed by aspiration. Then, the immunoprecipitation wash solution 2 was dispensed into each well at 200 (L/well, followed by aspiration. Then, the immunoprecipitation wash solution 3 was dispensed into each well at 200 (L/well, followed by aspiration.

After the washing, a Kinase reaction reagent was dispensed into each well at 50 (L/well, and stirred at 37(C at 900 rpm for 1 hour using a Plate Shaker (SI-300C, manufactured by AS ONE Corporation). The filter plate was taken out from the Plate Shaker and a recovery plate (manufactured by Bibby Sterilin Ltd.) was set on the lower part of the filter plate, followed by centrifugation at 4(C at 2000 rpm for 5 minutes using a centrifuge (AllegraTM 6KR Centrifuge, manufactured by Beckman Coulter, Inc.).

After the centrifugation, a reactant recovered on the recovery plate for activity measurement was transferred into each well of a reaction plate (manufactured by Applied Biosystem) at 14 (L/well and the fluorescent labeling reagent was dispensed into each well at 14 (L/well. After the dispensing, each solution was stirred under light shielding at 25(C at 400 rpm for 20 minutes using the Plate Shaker.

After the stirring, a fluorescent labeling reaction quenching solution was dispensed into each well of the reaction plate at 200 (L/well. The solution of each well of the reaction plate was transferred into each well of a measuring filter plate (manufactured by Millipore Corporation) at 100 (L/well. After aspiration of all the solutions for each well, the plate to be measured was cleaned by performing the washing process for dispensing the membrane wash solution into each well at 200 (L/well and aspirating each of the solutions twice. After the washing, the process for dispensing the fluorescence enhancing reagent into each well at 200 (L/well and aspirating each of the solutions 5 times, and the plate was dried.

After the drying, the fluorescence analysis of the plate to be measured is performed with the Plate Reader (Infinite F200, manufactured by Tecan Trading AG) and CDK1 and CDK2 activity values for each test sample were calculated based on the calibration curve. The calibration curve was prepared by subjecting three concentrations of solutions containing proteins (Recombinant CDK1/cyclin B1, active or Rccombinant CDK2/cyclin E, active) to fluorescence analysis in the above manner. 1 U (unit) of CDK1 activity and CDK2 activity to be measured means a value showing the fluorescence intensity equivalent to the fluorescence amount when the protein is 1 ng.

### <Measurement of expression levels of CDK1 and CDK2>

The reagents to be used were prepared in the following manner.
(1) Blocking reagent

| | |
|---|---|
| BSA | 10.0 g |
| 10x TBS solution | 25 mL |
| Ultrapure water | 212.8 g |
| 10% sodium azide aqueous solution | 2.25 mL |

(2) Membrane wash solution

| | |
|---|---|
| 10x TBS Solution | 100 mL |
| Ultrapure water | 900 mL |

(3) Anti-CDK1 antibody reagent

| | |
|---|---|
| Block Ace (manufactured by Dainippon Sumitomo Pharma Co., Ltd.) | 2.4 g |
| Ultrapure water | 72.3 g |
| 10% sodium azide aqueous solution | 750 (L |
| Anti-CDK1 antibody (0.455 mg/mL) (final concentration: 12 (g/mL) | 1.98 mL |

(4) Anti-CDK2 antibody reagent

| | |
|---|---|
| Block Ace (manufactured by Dainippon Sumitomo Pharma Co., Ltd.) | 2.4 g |
| Ultrapure water | 72.4 g |
| 10% sodium azide aqueous solution | 750 (L |
| Anti-CDK2 antibody (0.306 mg/mL) (final concentration: 7.5 (g/mL) | 1.94 mL |

(5) Secondary antibody reagent

| | |
|---|---|
| BSA | 2.5 g |
| 10x TBS solution | 25 mL |
| Ultrapure water | 216.3 g |
| 10% sodium azide aqueous solution (final concentration: 0.1%) | 2.21 mL |
| Biotinylated anti-rabbit IgG (manufactured by SouthernBiotech) (500 (g/mL, final concentration: 8 (g/mL) | 4.00 mL |

(6) Fluorescent labeling reagent

| | |
|---|---|
| BSA | 2.5 g |
| 10x TBS solution | 25 mL |
| Ultrapure water | 217.8 g |
| 10% sodium azide aqueous solution (final concentration: 0.1%) | 2.23 mL |
| Fluorescein-StreptAvidin (manufactured by Vector Laboratories)(1 mg/mL, final concentration: 10 (g/mL) | 2.50 ml |

(7) Fluorescence enhancing reagent

| | |
|---|---|
| 3-Morpholinopropanesulfonic acid (NW.209.25) (manufactured by DOJINDO LABORATORIES) | 41.9 g |
| Sodium azide | 2.0 g |
| 6N sodium hydroxide solution | 34 g |
| Ultrapure water | QS-2L |

(8) Test sample diluent (the first time)

| | |
|---|---|
| Membrane wash solution | 900 (L |
| 10% NP-40 Alternative PROTEIN GRADE (manufactured by CALBIOCHEM) | 100 (L |
| (9) Test sample diluent (the second time) Membrane wash solution | 19.9 mL |
| 10% NP-40 Alternative PROTEIN GRADE (manufactured by CALBIOCHEM) | 100 (L |

The expression levels of CDK1 and CDK2 were measured in the following manner.

Each test sample of the 29 types of breast cancer cell lines shown in Reference example 1 which had been prepared in the manner described in Reference example 2 was diluted so as to have a composition of 475 (L membrane wash solution, 300 (L test sample diluent, and 25 (L test sample. Then, the diluted test sample was dispensed into each well of the filter plate at 100 (L/well, followed by aspiration.

After the aspiration, the membrane wash solution was dispensed into each well of the filter plate at 300 (L/well, followed by aspiration and washing.

Then, the blocking was performed by dispensing the blocking reagent into each well of the filter plate at 100 (L/well and aspirating the blocking reagent.

A CDK1 antibody reagent and a CDK2 antibody reagent were dispensed into wells corresponding to CDK1 and CDK2 of the filter plate at 50 (L/well and each reagent was aspirated. Thereafter, the CDK1 antibody reagent and the CDK2 antibody reagent were again dispensed into each well at 50 (L/well and the plate was left at rest at 23(C for 2 hours on the Plate Shaker (SI-300C, manufactured by Tecan Trading AG) to react each reagent. Then, the reagent in each well was aspirated and the membrane wash solution was dispensed into each well at 300 (L/well, followed by aspiration and washing. The washing process was performed 4 times.

After the washing, wells corresponding to CDK1 and CDK2 of the filter plate were treated with a secondary antibody reagent in the same manner as the dispensing process of the CDK1 antibody reagent and the CDK2 antibody reagent to react the primary antibody with the secondary antibody, followed by washing. In this case, the settling time was set to 45 minutes and the washing process was performed twice.

The fluorescent labeling reagent was dispensed into each well of the filter plate at 100 (L/well, followed by aspiration. Further, the membrane wash solution was dispensed into each well at 300 (L/well, followed by aspiration and washing. The washing process was performed 4 times. Then, the fluorescence enhancing reagent was dispensed into each well of the filter plate at 200 (L/well and aspirated, and the plate was dried.

After the drying, the fluorescence analysis of the plate to be measured is performed with the Plate Reader (Infinite F200, manufactured by Tecan Trading AG) and CDK1 and CDK2 expression levels for each test sample were calculated based on the calibration curve. The calibration curve was prepared by subjecting four concentrations of solutions containing proteins (10% NP-40 Alternative PROTEIN GRADE (manufactured by CALBIOCHEM), CDK1/CyclinB1, active (manufactured by UPSTATE), or cdk2(1-298) (manufactured by Santa Cruz Biotechnology, Inc.)) to fluorescence analysis in the above manner.

### <Calculation of CDK1 and CDK2 specific activity values>

The CDK1 and CDK2 specific activity values were measured in the following manner. The CDK1 specific activity value (mU/ng) and the CDK2 specific activity value (mU/ng) were determined using the activity value and expression level of CDK1 and the activity value and expression level of CDK2 measured in the above-described manner by the following equation:
CDK1 specific activity value = CDK1 activity value/CDK1 expression level; and
CDK2 specific activity value = CDK2 activity value/CDK2 expression level.

Then, the value of CDK2 specific activity value/CDK1 specific activity value was calculated.

Here, the threshold value of the value of CDK2 specific activity value/CDK1 specific activity value was defined as 7.41, breast cancer cell lines having a value more than the threshold value were defined as cell lines with a high value of CDK2 specific activity value/CDK1 specific activity value, and breast cancer cell lines having a value less than the threshold value were defined as cell lines with a low value of CDK2 specific activity value/CDK1 specific activity value.

In this example, the CDK1 and CDK2 specific activity values of two cell lines of MDA-MB-415 and UACC812 among test samples of 29 types of breast cancer cell lines shown in Reference example 1 could not be calculated. Therefore, the results related to 27 types of breast cancer cell lines except for the two cell lines are shown in Fig. 7.

From the results shown in Fig. 7, among 27 types of breast cancer cell lines, cell lines with a high expression level of GST-π or a high value of CDK2 specific activity value/CDK1 specific activity value tend to have a high susceptibility to doxorubicin. As a result, it is shown that when the expression level of GST-π of breast cancer cells is high or when the value of CDK2 specific activity value/CDK1 specific activity value in breast cancer cells is higher than the threshold value, the susceptibility of breast cancer cells to an anthracycline anticancer agent can be judged as high. Further, cell lines with a low expression level of GST-π or a low value of CDK2 specific activity value/CDK1 specific activity value tend to have a low susceptibility to doxorubicin. As a result, it is shown that when the expression level of GST-π of breast cancer cells is low or when the value of CDK2 specific activity value/CDK1 specific activity value in breast cancer cells is lower than the threshold value, the susceptibility of breast cancer cells to an anthracycline anticancer agent can be judged as low.

### (Example 4)

### <Examination of effects of combination of expression levels of GST-π with value of CDK2 specific activity value/CDK1 specific activity value>

In accordance with the data obtained in Example 3, the effects of judgment based on the combination of the expression levels of GST-π with the value of CDK2 specific activity value/CDK1 specific activity value were examined using the judged results based on the expression level of GST-π alone, the judged results based on the value of CDK2 specific activity value/CDK1 specific activity value alone, and the judged results based on the combination of the expression levels of GST-π with the value of CDK2 specific activity value/CDK1 specific activity value.

### <Examination based on expression level of GST-π alone>

The results of the expression levels of GST-π and IC50 related to 27 types of breast cancer cell lines in Example 3 are shown in Table 2. Here, the threshold value of expression level of GST-π was defined as 8.05, breast cancer cell lines having a value more than the threshold value were defined as sensitive cell lines, and breast cancer cell lines having a value less than the threshold value were defined as nonsensitive cell lines.

In the results shown in Table 2, the odds ratio was 4.8, P according to logistic regression was 0.15, and AUC according to ROC analysis was 0.604.

**[Table 2]**

| Number of cases | | IC 50 (median) | | |
|---|---|---|---|---|
| | | (392 | >392 | Total |
| GST( | Nonsensitive | 12 | 10 | 22 (81.5) |
| | Sensitive | 1 | 4 | 5 (18.5) |
| | Total | 13 (48.1) | 14 (51.9) | 27 |

### <Examination based on value of CDK2 specific activity value/CDK1 specific activity value alone>

The results of the value of CDK2 specific activity value/CDK1 specific activity value and IC50 related to 27 types of breast cancer cell lines in Example 3 are shown in Table 3. Here, the threshold value of the value of CDK2 specific activity value/CDK1 specific activity value was defined as 13.0, breast cancer cell lines having a value more than the threshold value were defined as sensitive cell lines, and breast cancer cell lines having a value less than the threshold value were defined as nonsensitive cell lines.

In the results shown in Table 3, the odds ratio was 12.0, P according to logistic regression was 0.0117, and AUC according to ROC analysis was 0.712.

**[Table 3]**

| Number of cases | | IC 50 (median) | | |
|---|---|---|---|---|
| | | (392 | >392 | Total |
| CDK2 specific activity value/CDK1 specific activity value | Nonsensitive | 12 | 7 | 19(70.4) |
| | Sensitive | 1 | 7 | 8(29.6) |
| | Total | 13 (48.1) | 14 (51.9) | 27 |

### <Examination based on combination of expression level of GST-π and value of CDK2 specific activity value/CDK1 specific activity value>

The combination of the expression level of GST-π and the value of CDK2 specific activity value/CDK1 specific activity value and results of IC50 related to 27 types of breast cancer cell lines in Example 3 are shown in Table 4. Here, breast cancer cell lines in which the expression level of GST-π was more than 8.05 or the threshold value of the value of CDK2 specific activity value/CDK1 specific activity value was more than 13.0 were defined as sensitive cell lines, and breast cancer cell lines in which the threshold value of expression level of GST-π was less than 8.05 and the threshold value of the value of CDK2 specific activity value/CDK1 specific activity value was less than 13.0 were defined as nonsensitive cell lines.

In the results shown in Table 4, the odds ratio was 30.0, P according to logistic regression was 0.0004, and AUC according to ROC analysis was 0.819.

**[Table 4]**

| Number of cases | | IC 50 (median) | | |
|---|---|---|---|---|
| | | (392 | >392 | Total |
| combination | Nonsensitive | 12 | 4 | 16 (59.3) |
| | Sensitive | 1 | 10 | 11 (40.7) |
| | Total | 13 (48.1) | 14 (51.9) | 27 |

When the odds ratios corresponding to a risk ratio were compared according to Tables 2 to 4 and the obtained results, an odds ratio obtained by combining the expression level of GST-π and the value of CDK2 specific activity value/CDK1 specific activity value was higher than a value obtained summing each of the estimated individual odds ratios. This result suggests that judging performance of anthracycline susceptibility of breast cancer cells is synergistically improved by combining the expression level of GST-π and the value of CDK2 specific activity value/CDK1 specific activity value.

## Claims

1. A method for judging susceptibility of breast cancer cells contained in a biological sample to an anthracycline anticancer agent comprising steps of:
measuring expression levels of GST-π of breast cancer cells contained in a biological sample; and
judging the susceptibility of breast cancer cells contained in a biological sample to an anthracycline anticancer agent as high when the expression level of GST-π obtained by the measuring process is high.

2. The method according to claim 1, wherein
the judging step compares the expression level of GST-π obtained by the measuring step with the first threshold value and judges the susceptibility of breast cancer cells contained in a biological sample to an anthracycline anticancer agent as high when the expression level of GST-π is higher than the first threshold value.

3. The method according to any of claims 1 or 2, wherein
the anthracycline anticancer agent is daunorubicin, doxorubicin, pirarubicin, aclarubicin, epirubicin, oxaunomycin or idarubicin.

4. The method according to any of claims 1 to 3, wherein
the anthracycline anticancer agent is doxorubicin.

5. The method according to any of claims 1 to 4, wherein
the measuring step measures specific activity values of CDK1 and CDK2 contained in the biological sample containing breast cancer cells and
the judging step compares a value of specific activity value of CDK2 obtained by the measuring step/specific activity value of CDK1 obtained by the measuring step (CDK2 specific activity value/CDK1 specific activity value) with a second threshold value when the expression level of GST-π obtained by the measuring step is high, and the judging step judges the susceptibility of breast cancer cells contained in a biological sample to an anthracycline anticancer agent as high when the value of CDK2 specific activity value/CDK1 specific activity value is higher than the second threshold value.

6. The method according to claim 5, wherein
the judging step compares the expression level of GST-π obtained by the measuring step with the first threshold value and compares the value of CDK2 specific activity value/CDK1 specific activity value obtained by the measuring step with the second threshold value,
when the expression level of GST-π is higher than the first threshold value or when the value of CDK2 specific activity value/CDK1 specific activity value is higher than the second threshold value, the judging step judges the susceptibility of the breast cancer cells contained in a biological sample to an anthracycline anticancer agent as high.

7. The method according to claim 5, wherein
the judging step judges the susceptibility of breast cancer cells contained in a biological sample to an anthracycline anticancer agent as low when the expression level of GST-π obtained by the measuring step is low and the value of CDK2 specific activity value/CDK1 specific activity value is lower than the second threshold value.

8. The method according to claim 7, wherein
the judging step compares the expression level of GST-π obtained by the measuring step with the first threshold value and compares the value of CDK2 specific activity value/CDK1 specific activity value with the second threshold value, when the expression level of GST-π is lower than the first threshold value and the value of CDK2 specific activity value/CDK1 specific activity value is lower than the second threshold value, the judging step judges the susceptibility of breast cancer cells contained in a biological sample to an anthracycline anticancer agent as low when the expression level of GST-π obtained by the measuring step is low.

9. A computer program product comprising:
a computer readable medium, and
software instructions, on the computer readable medium, for enabling a computer to perform operations comprising:
acquiring the expression level of GST-π contained in a biological sample containing breast cancer cells;
comparing the expression level of GST-π with a first threshold value;
judging susceptibility of the breast cancer cells contained in the biological sample to an anthracycline anticancer agent as high when the expression level of GST-π is higher than the first threshold value; and
outputting the judged result.

10. The computer program product according to claim 9, wherein the operations further comprising:
calculating a value of specific activity value of CDK2/specific activity value of CDK1 (CDK2 specific activity value/CDK1 specific activity value) based on the activity value and expression level of CDK1 and the activity value and expression level of CDK2;
acquiring the activity value and expression level of CDK1 and the activity value and expression level of CDK2 in the biological sample containing breast cancer cells; and
comparing the value of CDK2 specific activity value/CDK1 specific activity value with a second threshold value;
wherein a judging operation judges the susceptibility of breast cancer cells contained in a biological sample to an anthracycline anticancer agent as high when the expression level of GST-π is higher than the first threshold value or when the value of CDK2 specific activity value/CDK1 specific activity value is higher than the second threshold value.

11. The computer program product according to claim 10,
wherein a judging operation judges the susceptibility of breast cancer cells contained in a biological sample to an anthracycline anticancer agent as low when the expression level of GST-π is lower than the first threshold value and the value of CDK2 specific activity value/CDK1 specific activity value is lower than the second threshold value.

12. The computer program product according to any of claims 9 to 11, wherein the anthracycline anticancer agent is daunorubicin, doxorubicin, pirarubicin, aclarubicin, epirubicin, oxaunomycin or idarubicin.

## Patentansprüche

1. Verfahren zum Beurteilen der Empfindlichkeit von in einer biologischen Probe enthaltenen Brustkrebszellen gegenüber einem Anthracyclin-Antikrebsmittel, umfassend die folgenden Schritte:
Messen des Expressionsgrades von GST-n von in einer biologischen Probe enthaltenen Brustkrebszellen; und
Beurteilen der Empfindlichkeit von in einer biologischen Probe enthaltenen Brustkrebszellen gegenüber einem Anthracyclin-Antikrebsmittel als hoch, wenn der mit dem Messvorgang erhaltene Expressionsgrad von GST-n hoch ist.

2. Verfahren nach Anspruch 1, wobei
der Beurteilungsschritt den mit dem Messschritt erhaltenen Expressionsgrad von GST-n mit dem ersten Schwellenwert vergleicht und die Empfindlichkeit von in einer biologischen Probe enthaltenen Brustkrebszellen gegenüber einem Anthracyclin-Antikrebsmittel als hoch beurteilt, wenn der Expressionsgrad von GST-n höher ist als der erste Schwellenwert.

3. Verfahren nach einem der Ansprüche 1 oder 2, wobei
es sich bei dem Anthracyclin-Antikrebsmittel um Daunorubicin, Doxorubicin, Pirarubicin, Aclarubicin, Epirubicin, Oxaunomycin oder Idarubicin handelt.

4. Verfahren nach einem der Ansprüche 1 bis 3, wobei es sich bei dem Anthracyclin-Antikrebsmittel um Doxorubicin handelt.

5. Verfahren nach einem der Ansprüche 1 bis 4, wobei
der Messschritt spezifische Aktivitätswerte von CDK1 und CDK2 misst, die in der Brustkrebszellen enthaltenden biologischen Probe enthalten sind, und
der Beurteilungsschritt den Wert eines mit dem Messschritt erhaltenen spezifischen Aktivitätswerts von CDK2/eines mit dem Messschritt erhaltenen spezifischen Aktivitätswerts von CDK1 ("spezifischer CDK2-Aktivitätswert/ spezifischer CDK1-Aktivitätswert") mit einem zweiten Schwellenwert vergleicht, wenn der mit dem Messschritt erhaltene Expressionsgrad von GST-n hoch ist, und der Beurteilungsschritt die Empfindlichkeit von in einer biologischen Probe enthaltenen Brustkrebszellen gegenüber einem Anthracyclin-Antikrebsmittel als hoch beurteilt, wenn der "spezifischer CDK2-Aktivitätswert/spezifischer CDK1-Aktivitätswert"-Wert höher als der zweite Schwellenwert ist.

6. Verfahren nach Anspruch 5, wobei
der Beurteilungsschritt den mit dem Messschritt erhaltenen Expressionsgrad von GST-n mit dem ersten Schwellenwert vergleicht und den mit dem Messschritt erhaltenen "spezifischer CDK2-Aktivitätswert/spezifischer CDK1-Aktivitätswert"-Wert mit dem zweiten Schwellenwert vergleicht,
der Beurteilungsschritt die Empfindlichkeit der in einer biologischen Probe enthaltenen Brustkrebszellen gegenüber einem Anthracyclin-Antikrebsmittel als hoch beurteilt, wenn der Expressionsgrad von GST-n höher als der erste Schwellenwert ist oder wenn der "spezifischer CDK2-Aktivitätswert/spezifischer CDK1-Aktivitätswert"-Wert höher ist als der zweite Schwellenwert.

7. Verfahren nach Anspruch 5, wobei
der Beurteilungsschritt die Empfindlichkeit von in einer biologischen Probe enthaltenen Brustkrebszellen gegenüber einem Anthracyclin-Antikrebsmittel als niedrig beurteilt, wenn der mit dem Messschritt erhaltene Expressionsgrad von GST-n niedrig ist und der "spezifischer CDK2-Aktivitätswert/spezifischer CDK1-Aktivitätswert"-Wert niedriger ist als der zweite Schwellenwert.

8. Verfahren nach Anspruch 7, wobei
der Beurteilungsschritt den mit dem Messschritt erhaltenen Expressionsgrad von GST-n mit dem ersten Schwellenwert vergleicht und den "spezifischer CDK2-Aktivitätswert/spezifischer CDK1-Aktivitätswert"-Wert mit dem zweiten Schwellenwert vergleicht, der Beurteilungsschritt die Empfindlichkeit von in einer biologischen Probe enthaltenen Brustkrebszellen gegenüber einem Anthracyclin-Antikrebsmittel als niedrig beurteilt, wenn der Expressionsgrad von GST-n niedriger als der erste Schwellenwert ist und der "spezifischer CDK2-Aktivitätswert/spezifischer CDK1-Aktivitätswert"-Wert niedriger ist als der zweite Schwellenwert.

9. Rechnerprogrammprodukt, umfassend:
ein rechnerlesbares Medium und
Softwareanleitungen auf dem rechnerlesbaren Medium, um einem Rechner die Durchführung von Rechenvorgängen zu ermöglichen, umfassend:
Erfassen des Expressionsgrades von GST-n, das in einer Brustkrebszellen enthaltenden biologischen Probe enthalten ist;
Vergleichen des Expressionsgrades von GST-n mit einem ersten Schwellenwert;
Beurteilen der Empfindlichkeit der in der biologischen Probe enthaltenen Brustkrebszellen gegenüber einem Anthracyclin-Antikrebsmittel als hoch, wenn der Expressionsgrad von GST-n höher ist als der erste Schwellenwert; und
Ausgeben des beurteilten Ergebnisses.

10. Rechnerprogrammprodukt nach Anspruch 9, wobei die Rechenvorgänge des Weiteren Folgendes umfassen:
Berechnen eines Werts eines spezifischen Aktivitätswerts von CDK2/eines spezifischen Aktivitätswerts von CDK1 ("spezifischer CDK2-Aktivitätswert/spezifischer CDK1-Aktivitätswert") ausgehend von dem Aktivitätswert und Expressionsgrad von CDK1 und dem Aktivitätswert und dem Expressionsgrad von CDK2; Erfassen des Aktivitätswerts und des Expressionsgrads von CDK1 und des Aktivitätswerts und des Expressionsgrads von CDK2 in der Brustkrebszellen enthaltenden biologischen Probe; und
Vergleichen des "spezifischer CDK2-Aktivitätswert/spezifischer CDK1-Aktivitätswert"-Werts mit einem zweiten Schwellenwert;
wobei ein Beurteilungsrechenschritt die Empfindlichkeit von in einer biologischen Probe enthaltenen Brustkrebszellen gegenüber einem Anthracyclin- Antikrebsmittel als hoch beurteilt, wenn der Expressionsgrad von GST-n höher ist als der erste Schwellenwert oder wenn der "spezifischer CDK2-Aktivitätswert/spezifischer CDK1-Aktivitätswert"-Wert höher ist als der zweite Schwellenwert.

11. Rechnerprogrammprodukt nach Anspruch 10,
wobei ein Beurteilungsrechenschritt die Empfindlichkeit von in einer biologischen Probe enthaltenen Brustkrebszellen gegenüber einem Anthracyclin-Antikrebsmittel als niedrig beurteilt, wenn der Expressionsgrad von GST-n niedriger ist als der erste Schwellenwert und der "spezifischer CDK2-Aktivitätswert/spezifischer CDK1-Aktivitätswert"-Wert niedriger ist als der zweite Schwellenwert.

12. Rechnerprogrammprodukt nach einem der Ansprüche 9 bis 11, wobei es sich bei dem Anthracyclin-Antikrebsmittel um Daunorubicin, Doxorubicin, Pirarubicin, Aclarubicin, Epirubicin, Oxaunomycin oder Idarubicin handelt.

## Revendications

1. Procédé d'évaluation de la susceptibilité de cellules de cancer du sein contenues dans un échantillon biologique à un agent anticancéreux anthracycline, ledit procédé comprenant les étapes consistant à :
mesurer les niveaux d'expression de la GST-π dans les cellules de cancer du sein contenues dans un échantillon biologique ; et
évaluer que la susceptibilité des cellules de cancer du sein contenues dans un échantillon biologique à un agent anticancéreux anthracycline est élevée lorsque le niveau d'expression de la GST-n obtenu par le procédé de mesure est élevé.

2. Procédé selon la revendication 1, dans lequel
l'étape d'évaluation compare le niveau d'expression de la GST-π obtenu par l'étape de mesure à la première valeur seuil et évalue que la susceptibilité des cellules de cancer du sein contenues dans un échantillon biologique à un agent anticancéreux anthracycline est élevée lorsque le niveau d'expression de la GST-π est supérieur à la première valeur seuil.

3. Procédé selon l'une quelconque des revendications 1 ou 2, dans lequel l'agent anticancéreux anthracycline est la daunorubicine, la doxorubicine, la pirarubicine, l'aclarubicine, l'épirubicine, l'oxaunomycine ou l'idarubicine.

4. Procédé selon l'une quelconque des revendications 1 à 3, dans lequel l'agent anticancéreux anthracycline est la doxorubicine.

5. Procédé selon l'une quelconque des revendications 1 à 4, dans lequel
l'étape de mesure mesure les valeurs d'activité spécifique des CDK1 et CDK2 contenues dans l'échantillon biologique contenant les cellules de cancer du sein et
l'étape d'évaluation compare une valeur du rapport valeur d'activité spécifique des CDK2 obtenue par l'étape de mesure/valeur d'activité spécifique des CDK1 obtenue par l'étape de mesure (valeur d'activité spécifique des CDK2/valeur d'activité spécifique des CDK1) à une seconde valeur seuil lorsque le niveau d'expression de la GST-n obtenu par l'étape de mesure est élevé, et l'étape d'évaluation évalue que la susceptibilité des cellules de cancer du sein contenues dans un échantillon biologique à un agent anticancéreux anthracycline est élevée lorsque la valeur du rapport valeur d'activité spécifique des CDK2/valeur d'activité spécifique des CDK1 est supérieure à la seconde valeur seuil.

6. Procédé selon la revendication 5, dans lequel
l'étape d'évaluation compare le niveau d'expression de la GST-π obtenu par l'étape de mesure à la première valeur seuil et compare la valeur du rapport valeur d'activité spécifique des CDK2/valeur d'activité spécifique des CDK1 obtenue par l'étape de mesure à la seconde valeur seuil,
lorsque le niveau d'expression de la GST-n est supérieur à la première valeur seuil ou lorsque la valeur du rapport valeur spécifique des CDK2/valeur d'activité spécifique des CDK1 est supérieure à la seconde valeur seuil, l'étape d'évaluation évalue que la susceptibilité des cellules de cancer du sein contenues dans un échantillon biologique à un agent anticancéreux anthracycline est élevée.

7. Procédé selon la revendication 5, dans lequel
l'étape d'évaluation évalue que la susceptibilité des cellules de cancer du sein contenues dans un échantillon biologique à un agent anticancéreux anthracycline est faible lorsque le niveau d'expression de la GST-π obtenu par l'étape de mesure est faible et la valeur du rapport valeur d'activité spécifique des CDK2/valeur d'activité spécifique des CDK1 est inférieure à la seconde valeur seuil.

8. Procédé selon la revendication 7, dans lequel
l'étape d'évaluation compare le niveau d'expression de la GST-π obtenu par l'étape de mesure à la première valeur seuil et compare la valeur du rapport valeur d'activité spécifique des CDK2/valeur d'activité spécifique des CDK1 à la seconde valeur seuil, lorsque le niveau d'expression de la GST-π est inférieur à la première valeur seuil ou lorsque la valeur du rapport valeur spécifique des CDK2/valeur d'activité spécifique des CDK1 est inférieure à la seconde valeur seuil, l'étape d'évaluation évalue que la susceptibilité des cellules de cancer du sein contenues dans un échantillon biologique à un agent anticancéreux anthracycline est faible lorsque le niveau d'expression de la GST-π obtenu par l'étape de mesure est faible.

9. Progiciel comprenant :
un support lisible par ordinateur, et
des instructions relatives au logiciel, sur le support lisible par ordinateur, destinées à permettre à un ordinateur de réaliser des opérations comprenant :
l'acquisition du niveau d'expression de la GST-n contenu dans un échantillon biologique contenant des cellules de cancer du sein ;
la comparaison du niveau d'expression de la GST-π à une première valeur seuil ;
le fait d'évaluer que la susceptibilité des cellules de cancer du sein contenues dans l'échantillon biologique à un agent anticancéreux anthracycline est élevée lorsque le niveau d'expression de la GST-π est supérieur à la première valeur seuil ; et
l'impression du résultat de l'évaluation.

10. Progiciel selon la revendication 9, dans lequel les opérations comprennent en outre :
le calcul d'une valeur du rapport valeur d'activité spécifique des CDK2/valeur d'activité spécifique des CDK1 (valeur d'activité spécifique des CDK2/valeur d'activité spécifique des CDK1) en se basant sur la valeur d'activité et le niveau d'expression des CDK1 ainsi que sur la valeur d'activité et le niveau d'expression des CDK2 ;
l'acquisition de la valeur d'activité et du niveau d'expression des CDK1 ainsi que de la valeur d'activité et du niveau d'expression des CDK2 dans l'échantillon biologique contenant les cellules de cancer du sein ; et
la comparaison de la valeur du rapport valeur d'activité spécifique des CDK2/valeur d'activité spécifique des CDK1 à une seconde valeur seuil ;
dans lequel une opération d'évaluation évalue que la susceptibilité des cellules de cancer du sein contenues dans un échantillon biologique à un agent anticancéreux anthracycline est élevée lorsque le niveau d'expression de la GST-π est supérieur à la première valeur seuil ou lorsque la valeur du rapport valeur d'activité spécifique des CDK2/valeur d'activité spécifique des CDK1 est supérieure à la seconde valeur seuil.

11. Progiciel selon la revendication 10,
dans lequel une opération d'évaluation évalue que la susceptibilité des cellules de cancer du sein contenues dans un échantillon biologique à un agent anticancéreux anthracycline est faible lorsque le niveau d'expression de la GST-π est inférieur à la première valeur seuil et la valeur du rapport valeur d'activité spécifique des CDK2/valeur d'activité spécifique des CDK1 est inférieure à la seconde valeur seuil.

12. Progiciel selon l'une quelconque des revendications 9 à 11, dans lequel l'agent anticancéreux anthracycline est la daunorubicine, la doxorubicine, la pirarubicine, l'aclarubicine, l'épirubicine, l'oxaunomycine ou l'idarubicine.
